# EUROPEAN PATENT SPECIFICATION

(11) **EP 1 720 641 B1**
(45) Date of publication and mention of the grant of the patent: **26.03.2014**
(21) Application number: 05710984.5
(22) Date of filing: 07.02.2005
(51) Int. Cl.: B01F 3/04, B01J 19/26, B01F 15/00

(54) **CYCLOHEXANE OXIDATION REACTOR AND PROCESS**
REAKTOR UND VERFAHREN ZUR OXIDIERUNG VON CYCLOHEXAN
REACTEUR ET PROCÉDÉ D'OXYDATION DU CYCLOHEXANE

(30) Priority: 17.02.2004 PL 36529904
(43) Date of publication of application: 15.11.2006
(73) Proprietor: Grupa Azoty S.A., 33-101 Tarnów (PL)
(72) Inventor: OCZKOWICZ, Stanislaw, PL-33-100 Tarnów (PL); RYGIEL, Stanislaw, 33-101 Tarnów (PL); WAIS, Jan, 33-101 Tarnów (PL); KOZIOL, Andrzej, 33-100 Tarnów (PL); GRUSZKA, Mateusz, 28-131 Solec Zdrój (PL); KONDRAT, Aleksandra, 33-100 Tarnów (PL); POHORECKI, Ryszard, 02-922 Warszawa (PL); WIERZCHOWSKI, Piotr, 04-743 Warszawa (PL); MONIUK, Wladyslaw, 02-904 Warszawa (PL); KRZYSZTOFORSKI, Andrzej, 33-100 Tarnów (PL); MIJAL, Witold, 33-100 Tarnów (PL); WOJCIK, Zbigniew, 33-100 Tarnów (PL); ZYLINSKI, Marek, 33-101 Tarnów (PL)
(74) Representative: Krawczyk, Lilianna
(86) International application number: PCT/PL2005/000009
(87) International publication number: WO 2005/077526

(56) References cited:
- DD-A5- 281 803
- US-B1- 6 432 149

## Description

The invention relates to the cyclohexane oxidation reactor and the process of cyclohexane oxidation mainly to cyclohexyl hydroperoxide.

Cyclohexane is oxidised in the liquid phase with oxygen-containing gases , usually at a temperature of 140 to 180°C in a reaction flow system, in the presence of a catalyst being a salt of a metal of variable valence. Because the selectivity of the oxidation process decreases with the increase of cyclohexane conversion, the conversion is kept at a low level, of about 3 - 4.5 %. This means large consumption of energy to recover unconverted cyclohexane and recirculate it to the oxidation process. In spite of keeping low conversion, selectivities higher than 78 - 82 % are rarely obtained. It means, that 18 - 22 % of the raw material is transformed into hardly useful or useless by-products, which involves additional cost of their utilisation. That's why, worldwide studies have been performed aimed at selectivity improvement of the cyclohexane oxidation process. Protection of the desired reaction products - namely cyclohexanone and cyclohexanol - from further oxidation to by-products is one of the directions. Application of boron acid, combining with cyclohexanol to form ester that is more resistant to oxidation than cyclohexanone and cyclohexanol, was the earliest effective solution. This method, although quite widely applied in the past, involved high investment and operating costs and was not easy to operate because of large quantities of solid to be recirculated. Finally, the savings resulting from the lower consumption of basic raw materials could hardly cover high expenditures.

The two stage cyclohexane oxidation process has been more widely used in the recent years. This process is based on a similar idea to keep products in an intermediate form more resistant to further oxidation, but without applying additional reagents and/or without operating with solids in the process. In this case it is cyclohexyl hydroperoxide that is the natural intermediate product in cyclohexane oxidation to cyclohexanone and cyclohexanol. In this process mainly cyclohexyl hydroperoxide is synthesised in the first stage by the reaction of cyclohexane with oxygen contained in the oxidising gas, and in the second stage this hydroperoxide is selectively decomposed into cyclohexanol and cyclohexanone. The two stage oxidation process is, among others, known from the patent descriptions: American US 5206441, European EP 0092867 and EP 0367326, as well as German No 3601218.

The Polish process of cyclohexane oxidation, called "Cyclopol" where the two-stage method is realized as described in the Polish patent description P-358357, goes in the same direction as well. In case of the Polish process, the two-stage method has to be adapted to the specific features of this process, e.g. to the multi-stage system of the catalytic oxidation reaction in a multi-sectional reactor, described mainly in the Polish patent description No 64449.

Because cyclohexyl hydroperoxide is more resistant to further oxidation than cyclohexanol and cyclohexanone thus, assuming the same good selectivity of cyclohexyl hydroperoxide decomposition, the higher the concentration of cyclohexyl hydroperoxide in the oxidation reaction product (oxidate), or - in other words - the higher the ratio of cyclohexyl hydroperoxide concentration to the total concentration of cyclohexanol and cyclohexanone, the more selective is the two-stage cyclohexane oxidation process. Usually, if the concentration of cyclohexyl hydroperoxide in the mixture leaving the oxidation reactor is higher than the total concentration of cyclohexanol and cyclohexanone, the concentration of cyclohexyl hydroperoxide can be considered high. But producing and keeping high concentration of cyclohexyl hydroperoxide (hereinafter shortly referred to as CHHP) is not easy. The known catalysts applied in the catalytic process usually increase both the rate of cyclohexane oxidation do CHHP (mainly by speeding-up initiation of the reaction, reducing the induction period, i.e. the period necessary to create sufficient concentration of alkyl and peroxyalkyl radicals) and CHHP decomposition to cyclohexanol and cyclohexanone (but selectivity of decomposition, when performed simultaneously with oxidation, i.e. in the presence of oxygen, is low). As concerns the non-catalytic process- first of all, such process is difficult to realise, as - even if no catalyst is added - small amount of ions dissolved in the reaction mixture due to slow corrosion of equipment in the corrosive reaction medium and - to a lesser degree - the metal surface of equipment, act as a catalyst increasing the rate of CHHP decomposition. The US patent description No 3510526 describes the method of protection from the catalytic action of the metal surface by its passivation with sodium pyrophosphate. Passivation of such surfaces to stop their catalytic action and make them corrosion-proof is effective, but it is also difficult to apply, expensive and of limited durability. The idea to apply other materials of construction for the reactor, e.g. titanium, for the same purpose as passivation is known as well. Application of titanium is very costly, especially in the solid form, the more because strength of titanium is lower than that of stainless steel; whereas reactor lining with titanium is difficult to manufacture. Moreover, according to several literature sources (e.g. Fiz. Mechanika Materialow, &(1), 16, 1971) the safety of titanium application in the medium of hydrocarbons oxidation reaction raises some doubts.

Secondly, even if the catalytic effects could be successfully excluded, the above mentioned problem with the induction period of the reaction would be an obstacle to run the process in a non-catalytic way. Lack of the catalytic effect would have to be compensated in another way, otherwise the process would require a long residence time, thus a large reaction volume would be necessary, which is both costly and hazardous in operation. Increasing the reaction temperature is the easiest way to compensate for the lack of catalyst. But a higher temperature increases the rate of CHHP decomposition, hence application of a non-catalytic process even at elevated temperature would not lead to a substantial increase of CHHP concentration in the oxidate. Temperature adjustment as a factor influencing the induction period is especially difficult in the autothermal "Cyclopol" process, in which the reaction heat is removed by cyclohexane evaporation; its rate is determined by pressure control being interrelated with temperature. The pressure in all sections of the reactor is the same, as the vapour spaces of all sections are connected by an off-gases collector.

There are chemical or physical methods of intervening in the process to reduce the induction period, increase the weight ratio of CHHP to the sum of cyclohexanol and cyclohexanone, or inhibit the decomposition of CHHP just formed in the reaction, finally to orient the catalytic action so as to, at least partially, limit the catalytic decomposition of CHHP but to maintain the advantageous influence of the catalyst on the induction period.

Autooxidation (initiation of the reaction chain) can be done by exposure to light (UV). This requires, however, the application of special catalysts, absorbing photon and transmitting the energy to the reacting molecule of the substrate. This method is still at the stage of laboratory studies, and the literature reports oxidation of hydrocarbons other than cyclohexane, e.g. izobutane to tertbutyl hydroperoxide with parallel conversion increase by application of zeolite catalyst (Chem Europe J., 2 (1996) 385). Oxidation of cyclic saturated hydrocarbons in liquid phase with molecular oxygen and under influence of photochemical activation has been described as well (Neftekhimiya 38 (1998) 22). Such methods are especially difficult from the technology point of view, as not only solid catalysts transparent to light, but also strong light sources having the defined characteristics are necessary. It would be especially difficult in case of cyclohexane oxidation as the cyclohexane-oxygen system poorly absorbs light, hence it would be necessary to apply special sensitising additives.

There are known methods of the induction period reduction where compounds forming radicals easier than cyclohexane are added to cyclohexane entering the reactor namely those having O-O bond cleavage energy below 75 kcal/mol, e.g. peroxides or hydroperoxides. For instance, in the US patent description 3948992 the addition of tertbutyl hydroperoxide with nitrogen oxide is described. Nitrogen dioxide, as an initiator, was studied in details by Berezin in the 1950s and 1960s; it was shown that it reacts with cyclohexane to form the alkyl radical. However, these additives should not react with CHHP or cyclohexane in reactions other than those belonging to the basic autooxidation chain. Thus the applicability of such additives has to be proved by a thorough study on a case-by-case basis. Generally, it is not recommended adding other (than those formed in the reaction) compounds to the reaction system, as some impurities could be introduced. On the other hand, neither cyclohexanol nor cyclohexanone can be used as a compound reacting with CHHP to form additional radicals branching the chain, as each of them would be further oxidised which is harmful to the selectivity. Thus the available room is rather narrow.

The method described in the US patent description No 4675450, aimed at maximisation of CHHP formation by preventing its decomposition, is based on application of complex esters of phosphoric acid, containing C₄ to C₁₂ alkyl radicals and C₅ to C₈ cycloalkyl radicals. Due to these additives, the catalyst is converted into a complex or a salt more polar than hydroperoxide, but also soluble in hydrocarbon at proper concentration, so as to prevent the hydrocarbon molecule from getting closer to the coordination sphere of the variable-valence metal cation, but still enabling the oxygen molecule to get closer. Such solution has some disadvantages, the two most essential are as follows. The addition of any polar ligands substantially decreases the catalyst solubility. Therefore a special treatment is necessary to obtain the suitable catalyst concentration , like that described in the US patent No 4341907, where the addition of pyridine is proposed, increasing the solubility of catalysts containing metal of variable valence combined with the alkyl phoshoric acid radical. Introduction of some heteroatoms-containing compounds (usually toxic) together with the catalyst results in the problems described above, namely product contamination with chemical compounds containing those atoms (phosphorous or nitrogen compounds), though in traces but still in a stable form (as compounds that contaminate cyclohexanone and cannot be completely removed in the downstream processes, from caprolactam to polyamide production). That means that the control of the final product quality might be lost.

A way is known of increasing CHHP yield in the cyclohexane oxidation reaction by application of additives increasing the concentration of alkyl and peroxyalkyl radicals (those from the main autooxidation chain). Tertbutyl alcohol is an example of such additive, applied as described in the US patent descriptions No 3987115 and 3949003. The main disadvantage of such solution is the necessary recirculation of large quantities of the added tertbutyl alcohol and purification of CHHP to remove the additive to a degree allowing CHHP to be easily decomposed in the further steps.

It has to be stressed that according to the state of the art relating to classical way of cyclohexane oxidation, the aim has usually been to reach as low as possible concentration of oxygen in off-gases. This resulted from both economical reasons (reduction of air consumption, whose compression requires energy) and for fear that too high a concentration of oxygen can lead to formation of an explosive cyclohexane - oxygen mixture). These problems were considered, e.g. in the Polish patent description No 144174, in the example relating to the process according to the invention the oxygen concentration reached 1.2 vol %.

From the analysis of the state of the art and the experience gathered, it results that most favourable for the selectivity would be the reaction run in a pure system, in which the natural induction period would not be decreased in a chemical way by adding the initiators, but by the proper engineering of the reactor. This means, first of all, a proper arrangement of the hydrodynamics and ensuring a well-organised flow of air and liquid. It is necessary to reach uniform gas distribution, nearly-ideal mixing of liquid, and especially avoid the formation of stagnant areas. If the process is run in a multi-sectional reactor, the aim is, of course, the ideal mixing within one section, while the reaction mixture flow through the successive sections is approximated by the plug-flow model of the reaction system. It is also essential, that the solution is correct from the point of view of oxygen absorption from the oxidising gas.

While improving the "Cyclopol" process, in the past lot of attention was paid to an engineering solution of the oxidation reactor aimed at optimisation of the flow dynamics of both liquid and gas. The Polish patent description No 94062 presented the protection from back flow of the liquid, i.e. protection from liquid flowing back to the previous section. The Polish patent No 136028 claimed such way of drilling of arc-shaped gas pipe distributors, that allows - in spite of the curvature - a uniform flow distribution of the oxidising gas above the distributor and a favourable liquid circulation. The Polish patent 147156 concentrated on the first section of the oxidation reactor, essential for the initiation of the oxidation reaction, and - taking into consideration the non-ideal mixing - claimed the multi-point introduction of fresh cyclohexane to this section. The Polish patent No 148651 described the velocity of air flow from the arc-shaped liquid distributors to prevent the possible cyclohexane ingress to the inside of the distributors. The Polish patent No 150660 claimed the direction and the velocity of air outflow from the distributors.

The Polish patent 152429 described the latest invention from this group, going deeply into the dynamics of gas and liquid flow in the reactor. This patent claimed the solution consisting in separating the neighbouring streams of air introduced into the reactor by the distributors (by applying long enough distance between the distributor arms and/or the baffles) by non-aerated areas, which should ensure an intensive multi-stage (within one section of the reactor) circulation around planes perpendicular to the liquid flow direction. The distance claimed in this patent was above 400 mm. It has to be added here that the multi-stage reaction run, i.e. liquid flow close to the plug flow, results from the rules of engineering of such chemical reactions when the desired products are intermediate products of the reaction which in the further run of the reaction are able to form by-products - thus mainly in the oxidation process, where oxidation and decomposition of CHHP proceed simultaneously. A process, in which mainly CHHP (more resistant to further oxidation than cyclohexanol and cyclohexanone) is desired, has different specificity, and the multi-stage character - or approaching the plug flow - is of less importance. It has been confirmed by investigation of the process macrokinetics and during the works on preparation of a mathematical model. The most essential then is the uniform aeration and nearly-ideal mixing in individual sections, which is favourable for the initiation of the reaction, namely creation of suitable concentration of free radicals (especially in the first or the initial section(s) of the reactor) and inhibits the non-selective decomposition of CHHP undesirable at this stage of the reaction which, in turn, is promoted by the non-uniform residence time of the mixture in the reactor, especially by the long residence time of CHHP in stagnant areas, or areas with limited mixing. Similarly, in the process aimed at maximisation of CHHP production, the fundamental phenomenon in the classical oxidation process - selectivity decrease with the increase of conversion - is less clear.

All patents relating to the engineering of oxidation reactor mentioned above were filed in the 1980s, when effective computer- aided tools of investigating and simulating the flow dynamics were not available. The calculation methods used then were labour-consuming and not very reliable; other methods applied in research of the "Cyclopol" process (like the isotopic investigation of flow) were very costly, thus their application was very limited. Only in the recent years, after elaboration and application of new mathematical methods like CFD (computational fluid dynamics), numerically solving equations describing the momentum and energy and mass balances, new possibilities have become available, allowing complex and exhaustive research on hydrodynamics of cyclohexane oxidation reactor to be started. This work consisted not only in theoretical studies, simulations and modelling of the flow. Basing on them the reactor internals have been modernised and the results, relating to the degree of oxygen absorption, direction of process run and process selectivity, have been tested on a commercial scale. It has been found that - although the above mentioned patents constituted essential progress in that time - now some of the observations and conclusions presented there need to be verified and revised. It concerns the most technically advanced solutions claimed in the patent No 152429 as well. It has been found that the multi-stage circulation within one section does not ensure well-ordered flow of gas and liquid. The flow happened to be chaotic, characterized by areas with very differentiated distribution of liquid velocity (including stagnant areas) and gas hold-up in the gas-liquid mixture. The large distance between the distributor arms (and thus lower number of the arms) resulted in a concentrated and intensive gas flow. The gas bubbles velocities were too high reducing the degree of oxygen absorption (oxygen "breakthrough" to the vapour area). It has been found as well that from the two systems of baffles presented in the mentioned patent description, the system with a single baffle between each pair of arms ensures more orderly flow pattern than that of two baffles preferred in the patent. Using no baffles markedly impairs the mixing process (among other things, because liquid flow alongside the reactor disturbs the upward flow of gas bubbles). Moreover, it has been found that the height of baffles (the height has not been defined in the patent 152429) and their shape (only schematically marked on the drawing in this patent) are essential for the flow pattern.

Finally, as a result of the said research, it has been found that the solution of the oxidation reactor can be essentially improved, ensuring a uniform distribution of gas in the oxidation mixture, close-to-ideal liquid mixing in each section, a uniform distribution of the liquid velocity and non-existence of stagnant areas, thus facilitating the initiation of the reaction and inhibiting the decomposition of CHHP, all this keeping good oxygen absorption, without oxygen "breakthrough" to the vapour space of the reactor. According to the invention, the engineering solution of the reactor consists in placing arc-shaped gas distributors at a short distance to each other, that is best described as the multiplicity of the outside diameter of the pipe distributor. The ratio of the distance between the distributors to the pipe diameter is in the range from 2 to 4, preferably from 2.5 to 3.5. Between each pair of the distributor arms is located one vertical baffle having the shape of a segment of a circle with vertically cut ends, whose height (in relation to the arrow of the distributor arm arc, i.e. the vertical distance between the lowest and highest point of the arc) has been selected so as to obtain the optimum bubbling layer and undisturbed vertical flow of gas bubbles. The ratio of such height to the arrow of the arc is in the range from 0.5 to 1.25, preferably from 0.6 to 1.0, and the lower, semicircular edge of the baffle shall be placed at the elevation of the respective arc-shaped pipe elements with the matching curvature, i.e. fit the end view of the latter.

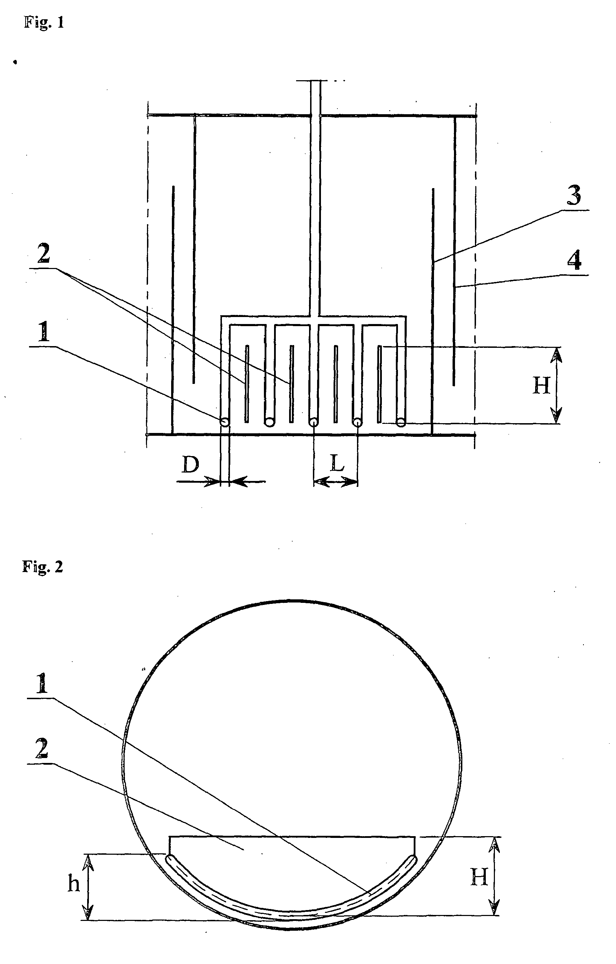

A reactor according to the invention, a horizontal vessel divided by a system of partitions into sections, is shown as an example on the drawing of one reactor section whose longitudinal and cross sections are shown in Fig. 1 and Fig. 2, respectively. In each section pipe distributors are placed, consisting of drilled arc-shaped elements 1, connected by a system of collectors with oxidising gas inlet from outside of the reactor. The diameter D of the drilled pipe equals 100 mm. The distance L between the axes of individual pipe elements is 300 mm. The directional baffles 2 having the shape of a segment of a circle with vertically cut ends are placed between the arc-shaped elements. The lower, semicircular edge of the baffle is placed at the same level, as the corresponding arc-shaped pipe element of the distributor, it means it fits the end view of this pipe element 1. The baffle height H is 10 % higher than the arrow h of the arc-shaped element. The section is separated from the adjacent sections by partitions 3 and 4.

After realisation of the above new engineering solution of internals of the cyclohexane oxidation reactor, it turned out that the problem of obtaining oxidate with high concentration ratio of CHHP to cyclohexanone plus cyclohexanol, or with high concentration of CHHP, was solved. The solution according to the invention allows that objective to be reached in an effective, relatively simple way, in conformity with the specific features of the "Cyclopol" process, avoiding disadvantages and insufficiencies of solutions as per the state of the art. The above mentioned inconveniences of the non- catalytic process could be avoided by combining in a complementary way the reactor solution with technological measures, without having to apply complicated and expensive catalysts, especially those containing heteroatoms that could adversely influence product purity. Typical catalysts, namely the salts of metals of variable valence, are sufficient for the solution described. It is not necessary to apply other (besides the catalyst) components, which are not formed in the process. This also helps keep high quality of the final product. Neither non-standard and expensive materials of construction nor the difficult passivation of the commonly used austenitic steels have to be applied.

Unexpectedly it was found, that the mentioned result could be obtained while running the process in the reactor described above by keeping a defined concentration of oxygen in off-gases, higher than that according to the state of the art, and keeping a low catalyst concentration, in a precisely defined range. The solution according to the invention is supported by tests on both a small and, mainly, on a commercial scale. Basing on these tests it has been found that during the cyclohexane oxidation process mainly to cyclohexyl hydroperoxide in the liquid phase, under pressure, with oxygen containing gases, at a temperature of 155 to 170 °C, in a multi-sectional flow-through reactor being a horizontal vessel divided into sections by pairs of partitions, where pipe distributors of oxygen-containing gas are placed, consisting of drilled arc-shaped elements connected with collectors supplying the oxidising gas from the outside of the reactor, and single directional baffles are placed between the arc-shaped elements, in the presence of a homogenous catalyst being the salt of a metal with variable valence , the average concentration of catalyst should be kept at the level above 0.05 ppm but below 0.1 ppm of metal in relation to cyclohexane introduced into the reactor, and the average concentration of oxygen in off-gases (i.e. gases in the collector at the gas outlet from the reactor) on a dry-gas basis (without considering cyclohexane the gases are saturated with) should not be lower than 2.0 vol. %, it should be kept in the range from 2.0 to 3.0 %, preferably in the range from 2.2. to 2.5 %, while in the cyclohexane oxidation reactor the ratio of the distance (L) between the arc-shaped pipe elements (1) to the pipe diameter (D) is in the range from 2 to 4, the directional baffles (2) having the shape of a segment of a circle with vertically cut ends and their lower, semicircular edges are located in such way that they fit the end view of the arc-shaped elements (1), and the ratio of the baffle height (H) to the arrow (h) of the arc-shaped element is in the range from 0.5 to 1.25. It is especially essential to keep at least the same, preferably higher, in the range from 2.4 to 2.8 %, oxygen concentration in off-gases from the first and second sections of the reactor. The technological progress done since the said patent No 144174 was filed and the present detailed safety analyses such as the "hazop study" lead to the conclusion that the above range of oxygen concentration is absolutely safe. The mechanism of influence of the increased oxygen concentration in off gases on the increased concentration of CHHP has not yet been theoretically explained in a convincing way, among other things, because of the above mentioned specificity of the cyclohexane oxidation process mainly to CHHP (basically different from the conventional process involving simultaneous oxidation of cyclohexane and decomposition of CHHP), novelty of this first process and still insufficient knowledge of its macrokinetics. It is known that oxygen concentration in off-gases depends on the concentration of oxygen dissolved in the liquid phase or in the interphase layer. The concentration of oxygen in off-gases reflects the phenomena occurring in the process, thus can be considered a representative, useful, and, especially, easily measurable criterion of operating the process. It has to be stressed that running the process basing on oxygen concentration in off-gases is possible provided that a reactor according to the invention is applied, since one has to exclude the occurrence of increased oxygen concentration in off-gas due to - only or partially - an incorrect engineering solution, e.g. allowing oxygen to "breakthrough", or due to a deviation from the proper process run being known to an expert in this field (e.g. deviation from the required temperature).

The following examples describe the process according to the invention, without limiting its scope. Example 1 describes the process without using the method according to the invention, Example 2 describes the process according to the invention.

### Example 1.

The cyclohexane oxidation process was performed in a six-sectional flow-trough reactor having the construction known from the Polish patent description No 152429. The reactor was charged with 160 tons per hour of cyclohexane , and air was introduced to each of its sections, totally 6200 Nm³ per hour. The catalyst being a salt of a metal with variable valence (cobalt octoate) was added to the first section in such quantity, that the average metal concentration in the reaction liquid was 0.5 ppm. Process temperature of 160 - 165 °C and pressure of 0.9 to 1.05 MPa were kept; thus the average oxygen concentration in off-gases reached 1.5 vol %. 4360 kg/h of cyclohexane were reacted. Oxidate leaving the reactor contained totally 4140 k/h of the desired products (cyclohexanol, cyclohexanone and CHHP) The weight ratio of CHHP to the sum of other desired products was equal to 0.14. The selectivity of the process to the desired products reached the level of 81 %.

### Example 2

The cyclohexane oxidation process was performed in a a six-sectional flow-through reactor having the construction according to this invention. The same amounts of cyclohexane and air were added to the reactor as in Example 1. To the first section the same catalyst was added as in Example 1, but in such quantity, that the average metal concentration in the reaction liquid was 0.08 ppm. The process temperature and pressure were the same as in Example 1. The process was operated in such a way that the average oxygen concentration in off-gases was equal to 2.4 vol % , and the oxygen concentration in off-gases leaving the first and second sections reached 2.7 vol % . 4400 kg/h of cyclohexane were reacted. Oxidate leaving the reactor contained totally 4610 k/g of the desired products (cyclohexanol, cyclohexanone and CHHP) The weight ratio of CHHP to the sum of other desired products was 0.39. The selectivity of the process to the desired products reached the level of 88 %.

### Example 3

A reactor according to the invention, a horizontal vessel divided by a system of partitions into sections, is shown as an example on the drawing of one reactor section whose longitudinal and cross sections are shown in Fig. 1 and Fig. 2, respectively. In each section pipe distributors are placed, consisting of drilled arc-shaped elements 1, connected by a system of collectors with oxidising gas inlet from outside of the reactor. The diameter D of the drilled pipe equals 100 mm. The distance L between the axes of individual pipe elements is 300 mm. The directional baffles 2 having the shape of a segment of a circle with vertically cut ends are placed between the arc-shaped elements. The lower, semicircular edge of the baffle is placed at the same level, as the corresponding arc-shaped pipe element of the distributor, it means it fits the end view of this pipe element 1. The baffle height H is 10 % higher than the arrow h of the arc-shaped element. The section is separated from the adjacent sections by partitions 3 and 4.

## Claims

1. A reactor for cyclohexane oxidation, mainly to cyclohexyl hydroperoxide, operating in a flow-through system, being a horizontal vessel divided into sections by pairs of partitions, where pipe distributors of oxygen-containing gas are placed, consisting of drilled arc-shaped elements connected with collectors supplying the oxidising gas from the outside of the reactor, and single directional baffles are placed between the arc-shaped elements, **in which** the ratio of the distance (L) between the arc-shaped pipe elements (1) to the pipe diameter (D) is in the range from 2 to 4, preferably from 2.5 to 3.5, the directional baffles (2) having the shape of a segment of a circle with vertically cut ends and their lower, semicircular edges are located in such way that they fit the end view of the arc-shaped elements (1), and the ratio of the baffle height (H) to the arrow (h) of the arc-shaped element is in the range from 0.5 to 1.25, preferably from 0.6 to 1.

2. A process for cyclohexane oxidation, mainly to cyclohexyl hydroperoxide, in the liquid phase, under pressure, with oxygen-containing gases, at a temperature of 155 to 170 °C, in a multi-sectional flow-through reactor, being a horizontal vessel divided into sections by pairs of partitions, where pipe distributors of oxygen-containing gas are placed, consisting of drilled arc-shaped elements connected with collectors supplying the oxidising gas from the outside of the reactor, and single directional baffles are placed between the arc-shaped elements, in the presence of a homogenous catalyst being the salt of a metal with variable valence, **in which** the ratio of the distance (L) between the arc-shaped pipe elements (1) to the pipe diameter (D) is in the range from 2 to 4, the directional baffles (2) having the shape of a segment of a circle with vertically cut ends and their lower, semicircular edges are located in such way that they fit the end view of the arc-shaped elements (1), and the ratio of the baffle height (H) to the arrow (h) of the arc-shaped element is in the range from 0.5 to 1.25 and the average concentration of metal in the reaction liquid is kept at the level above 0.05 ppm but below 0.1 ppm, and the average concentration of oxygen in off-gases, on a dry off-gas basis, is in the range from 2.0 to 3.0 % by volume, preferably from 2.2. to 2.5 % by volume.

3. The process according to claim 2, in which the oxygen concentrations in off-gases from the first and second sections are at the level from 2.2 to 3.0 % by volume, preferably from 2.4 to 2.7 % by volume.

## Patentansprüche

1. Ein Reaktor für Cyclohexan-Oxidation, hauptsächlich zu Cyclohexyl-Wasserstoffperoxid, der mit einem Durchflusssystem funktioniert und der aus einem durch paarweise Abteilungen in vier Sektionen gegliederten horizontalen Behälter besteht, Rohrverteiler mit sauerstoffhaltigem Gas angebracht sind, die aus gebohrten, kreisbogenförmigen Elementen bestehen, welche mit Kollektoren verbunden sind, die das oxidierende Gas von außen dem Reaktor zuführten, sowie unidirektionale Sperrungen zwischen den kreisbogenförmigen Elementen angebracht sind, **wobei** denen das Verhältnis des Abstands (L) zwischen den kreisbogenförmigen Rohrelementen (1) zum Rohrdurchmesser (D) im Bereich von 2 bis 4 liegt, vorzugsweise zwischen 2,5 und 3,5, wobei die Richtungssperrungen (2) die Form eines Kreissegments mit vertikal geschnittenem Ende besitzen und ihre tiefer liegenden, halbkreisförmigen Enden so angebracht sind, dass sie zum endseitigen Aussehen der kreisbogenförmigen Elemente (1) passen und das Verhältnis der Höhe der Sperrungen (H) zur gerichteten Kante (h) des kreisbogenförmigen Elements im Bereich von 0,5 bis 1,25, vorzugsweise zwischen 0,6 und 1 liegt.

2. Ein Prozess zur Cyclohexan-Oxidation, hauptsächlich zu Cyclohexyl-Wasserstoffperoxid, in flüssiger Phase unter Druck mit sauerstoffhaltigen Gasen bei einer Temperatur von 155 bis 170°C, in einem mehrteiligen Durchflussreaktor, der aus einem durch paarweise Abteilungen in Sektionen gegliederten horizontalen Behälter besteht, wo Rohrverteiler mit sauerstoffhaltigem Gas angebracht sind, die aus gebohrten, kreisbogenförmigen Elementen bestehen, welche mit Kollektoren verbunden sind, die das oxidierende Gas von außen dem Reaktor zuführen, sowie unidirektionale Sperrungen zwischen den kreisbogenförmigen Elementen angebracht sind, in Gegenwart eines homogenen Katalysators in Form des Salzes eines Metalls mit wechselnder Valenz, **wobei** das Verhältnis des Abstands (L) zwischen den kreisbogenförmigen Rohrelementen (1) zum Rohrdurchmesser (D) im Bereich von 2 bis 4 liegt, die Richtungssperrungen (2) die Form eines Kreissegments mit vertikal geschnittenem Ende besitzen und ihre tiefer liegenden, halbkreisförmigen Enden so angebracht sind, dass sie zum endseitigen Aussehen der kreisbogenförmigen Elemente (1) passen, das Verhältnis der Höhe der Sperrungen (H) zur gerichteten Kante (h) des kreisbogenförmigen Elements im Bereich von 0,5 bis 1,25 liegt, die durchschnittliche Metallkonzentration in der Reaktionsflüssigkeit auf einem Niveau von über 0,05 ppm, aber unter 0,1 ppm gehalten wird und die durchschnittliche Sauerstoffkonzentration in den Abgasen bei trockenen Abgasen im Bereich von 2,0 bis 3,0 Volumenprozent, vorzugsweise zwischen 2,2 und 2,5 Volumenprozent liegt.

3. Der Prozess gemäß Anspruch 2, **wobei** die Sauerstoffkonzentration in den Abgasen aus der ersten und zweiten Sektion im Bereich von 2,2 bis 3,0 Volumenprozent, vorzugsweise zwischen 2,4 und 2,7 Volumenprozent liegt.

## Revendications

1. Un réacteur pour l'oxydation du cyclohexane, principalement de l'hydroperoxyde de cyclohexyle, qui opère dans un système d'écoulement permanent constituant le réceptacle horizontal, est divisé en sections par des cloisons paires et où se trouvent les conduites qui distribuent le gaz contenant de l'oxygène, constituées d'éléments perforés en forme d'arc, connectées aux collecteurs de distribution du gaz oxydant de l'extérieur du réacteur et les déflecteurs unidirectionnels situés entre les éléments en forme d'arc, **caractérisé en ce que** le rapport de la distance (L) entre les conduites en forme d'arc (1) et le diamètre de la conduite (D) est de 2 à 4, de préférence de 2,5 à 3,5, les déflecteurs directionnels (2) ont la forme d'un segment d'un cercle avec ses extrémités coupées verticalement et ses bords semi-circulaires sont placés de telle façon qu'ils s'adaptent à la vue latérale des éléments en forme d'arc (1) où le rapport entre la hauteur (H) du déflecteur et la flèche (h) de l'élément en forme d'arc est de 0,5 à 1,25, de préférence de 0,6 à 1.

2. Un processus pour l'oxydation du cyclohexane, principalement de l'hydroperoxyde de cyclohexyle, dans la phase liquide, sous pression, avec du gaz contenant de l'oxygène, à une température de 155 à 170°C dans un réacteur multi-section d'écoulement permanent, constituant le réceptacle horizontal est divisé en sections par de cloisons paires et où se trouvent les conduites qui distribuent le gaz contenant de l'oxygène, constituées d'éléments perforés en forme d'arc, connectées aux collecteurs de distribution du gaz oxydant de l'extérieur du réacteur et les déflecteurs unidirectionnels situés entre les éléments en forme d'arc (1), en présence d'un catalyseur homogène qui est le sel d'un métal avec une valence variable **caractérisé en ce que** le rapport de la distance (L) entre les conduites en forme d'arc (1) et le diamètre de la conduite (D) est de 2 à 4, les déflecteurs directionnels (2) ont la forme d'un segment d'un cercle avec ses extrémités coupées verticalement et ses bords semi-circulaires sont placés de telle façon qu'ils s'adaptent à la vue latérale des éléments en forme d'arc (1) où le rapport entre la hauteur (H) du déflecteur et la flèche (h) de l'élément en forme d'arc est de 0,5 à 1,25 et la concentration moyenne de métal dans le liquide de réaction est maintenue à un niveau au-dessus de 0,05 ppm, mais inférieure à 0,1 ppm et la concentration moyenne d'oxygène dans les effluents gazeux, sur une base d'effluents de gaz sec, est de 2,0 à 3,0% par volume, de préférence de 2,2 à 2,5% par volume.

3. Le processus selon la revendication 2, **caractérisé en ce que** les concentrations de gaz dans les effluents gazeux de la première et la deuxième section décrivent un arc à un niveau de 2,2 à 3,0 % par volume, de préférence de 2,4 à 2,7 % par volume.
